# EUROPÄISCHE PATENTANMELDUNG

(11) **EP 1 389 452 A2**
(43) Veröffentlichungstag der Anmeldung: **18.02.2004**
(21) Anmeldenummer: 03014892.8
(22) Anmeldetag: 01.07.2003
(51) Int. Cl.: A61F 2/38

(54) **Kniegelenkprothese**

(30) Priorität: 16.08.2002 DE 10237444
(71) Anmelder: Igel, Hanns-Jörg, 66440 Bierbach (DE)
(72) Erfinder: Igel, Hanns-Jörg, 66440 Bierbach (DE)
(74) Vertreter: Patentanwälte Hauck, Graalfs, Wehnert, Döring, Siemons, Schildberg

(57) **Zusammenfassung**

Es wird eine Kniegelenkprothese mit einer Femurkomponente, einer Tibiakomponente mit einer Plattform und einem zwischen der Femur- und Tibiakomponente angeordneten Lagereinsatz beschrieben. Zwischen der Plattform der Tibiakomponente und dem Lagereinsatz befindet sich ein Verbindungsteil zur drehbaren Lagerung des Lagereinsatzes relativ zur Plattform. Tibiakomponente und Lagereinsatz haben je eine Ausnehmung zur Aufnahme des Verbindungsteiles, wobei das Verbindungsteil aus einer von der Ausnehmung der Tibiakomponente oder des Lagereinsatzes aufgenommenen Stellung in eine in die andere Ausnehmung hineinragende und ein Drehlagerelement für den Lagereinsatz bildende Stellung und wieder zurückbewegbar ist. Auf diese Weise läßt sich der Lagereinsatz bei implantierter Kniegelenkprothese in besonders einfacher Weise entfernen bzw. austauschen.

## Beschreibung

Die vorliegende Erfindung betrifft eine Kniegelenkprothese mit einer Femurkomponente, einer Tibiakomponente mit einer Plattform und einem zwischen der Femur- und Tibiakomponente angeordneten Lagereinsatz, wobei sich von der Plattform der Tibiakomponente ein Verbindungsteil in Richtung des Lagereinsatzes erstreckt und der Lagereinsatz eine Ausnehmung zur Aufnahme des Verbindungsteiles zur drehbaren Lagerung des Lagereinsatzes aufweist.

Eine derart ausgebildete Kniegelenkprothese ist beispielsweise aus der EP 0 678 286 B1 bekannt. Bei der bekannten Prothese ist das Verbindungsteil als sich von der Plattform der Tibiakomponente aus erstreckender und hiermit einstükkig ausgebildeter ringförmiger Flansch ausgebildet, und der Lagereinsatz weist an seiner Unterseite eine Ausnehmung zur drehbaren Lagerung des Lagereinsatzes auf dem Flansch der Tibiakomponente auf. Dieser Flansch besitzt an seiner Außenseite mindestens eine Nut, und die Innenwand der Ausnehmung hat mindestens eine Rippe, die sich zumindest entlang eines Teiles des Innenumfanges der Ausnehmung erstreckt und in die Nut eingreift. Auf diese Weise wird eine Kniegelenkprothese geschaffen, bei der eine verschleiß- und reibungsarme Rotation zwischen dem Lagereinsatz und der Tibiakomponente und eine Arretierung des Lagereinsatzes in Richtung auf die Femurkomponente mit einfachen Mitteln gewährleistet ist.

Um bei einer derartigen Kniegelenkprothese im implantierten Zustand derselben einen Austausch des Lagereinsatzes, der in der Regel aus einem hierfür geeigneten Kunststoff ausgebildet ist, während die Femur- und Tibiakomponente aus Metall bestehen, zu ermöglichen, müßten Femurkomponente und Tibiakomponente mit Lagereinsatz voneinander getrennt werden, wonach der Lagereinsatz vom Flansch der Plattform der Tibiakomponente abgezogen werden kann, wozu ausreichend Raum für den Operateur zur Verfügung stehen muß. Danach kann ein neuer Lagereinsatz auf den Flansch aufgeclipst werden. Es versteht sich, daß ein solcher Austausch des Lagereinsatzes aufwendig und kompliziert ist, da dieser nur in Axialrichtung von der Plattform der Tibiakomponente gelöst werden kann. Dies ist aber bei den zur Verfügung stehenden beschränkten räumlichen Verhältnissen nur schwer durchzuführen.

Der vorliegenden Erfindung liegt die Aufgabe zugrunde, eine Kniegelenkprothese der angegebenen Art zu schaffen, bei der sich, im implantierten Zustand der Prothese, der Lagereinsatz in besonders einfacher Weise entfernen bzw. austauschen läßt.

Diese Aufgabe wird erfindungsgemäß bei einer Kniegelenkprothese der angegebenen Art dadurch gelöst, daß die Tibiakomponente mit Plattform eine Ausnehmung zur Aufnahme des Verbindungsteiles aufweist und daß das Verbindungsteil in die Ausnehmung versenkbar ist.

Bei den hier in Rede stehenden Kniegelenkprothesen ist es erwünscht, den Lagereinsatz in Abhängigkeit von den anatomischen Gegebenheiten, beispielsweise bei fortschreitender Insuffizienz des Bandapparates, austauschen zu können, um beispielsweise durch Einbau eines anderen Lagereinsatzes eine bessere Führung der Femurkomponente zu erreichen. Ferner tritt im Laufe der Zeit ein Verschleiß des vorzugsweise aus einem geeigneten Kunststoff, insbesondere Polyäthylen, bestehenden Lagereinsatzes auf, so daß dieser ausgetauscht werden muß. Man ist daher bestrebt, bei implantierter Kniegelenkprothese eine einfache Entfernung bzw. einen einfachen Austausch des Lagereinsatzes zu erreichen.

Wie vorstehend ausgeführt, ist dies bei der vorstehend beschriebenen Kniegelenkprothese des Standes der Technik mit Schwierigkeiten verbunden. Ähnliche Schwierigkeiten treten auf, wenn der Lagereinsatz einen sich nach unten erstrekkenden Drehzapfen aufweist, der in einen sich verjüngenden, hohlen Zapfen an der Unterseite der Tibiakomponente eingreift. Auch hier muß der Lagereinsatz in Axialrichtung von der Tibiakomponente entfernt werden, da er mit dem sich nach unten erstreckenden Drehzapfen versehen ist. Bei anderen Lösungen mit entsprechenden, fest angebrachten Verbindungsteilen bzw. Verbindungsansätzen treten vergleichbar Probleme auf.

Mit der erfindungsgemäßen Lösung wird hierfür Abhilfe geschaffen. Da hierbei das Verbindungsteil weder einstückig mit der Tibiakomponente oder dem Lagereinsatz ausgebildet noch an diesen befestigt ist, sondern ein hiervon unabhängiges Teil bildet, kann es in eine von der Ausnehmung der Tibiakomponente oder des Lagereinsatzes aufgenommene Stellung gebracht werden, in der es nicht von unten aus dem Lagereinsatz oder von oben von der Plattform der Tibiakomponente herausragt, so daß in dieser Stellung der Lagereinsatz in Anterior- oder Posterior-Richtung aus der implantierten Kniegelenkprothese herausgenommen werden kann, ohne daß hierbei der Lagereinsatz von der Tibiakomponente in tibiaparalleler Richtung wegbewegt werden muß. Wenn der Lagereinsatz entfernt und ein neuer Lagereinsatz eingesetzt worden ist, wird das Verbindungsteil aus seiner von der Ausnehmung der Tibiakomponente oder des Lagereinsatzes aufgenommenen Stellung in eine in die andere Ausnehmung hineinragende Stellung gebracht, in der es ein Drehlagerelement für den Lagereinsatz bildet, so daß sich einerseits der Lagereinsatz um das Drehlagerelement drehen kann oder andererseits sich der Lagereinsatz mit dem Verbindungsteil relativ zur Plattform der Tibiakomponente drehen können.

Das Verbindungsteil steht daher in seiner Funktionsstellung entweder vom Lagereinsatz nach unten oder von der Plattform der Tibiakomponente nach oben vor, während es in seiner Stellung zum Entfernen oder Austauschen des Lagereinsatzes in der Ausnehmung des Lagereinsatzes oder in der Ausnehmung der Tibiakomponente aufgenommen ist, so daß sich der Lagereinsatz bequem in Richtungen senkrecht zur Tibiaachse (Anterior- oder Posterior-Richtung) entfernen läßt.

Die Bewegung des Verbindungsteiles zwischen dessen beiden Endstellungen wird vorzugsweise mit Hilfe eines hierfür geeigneten Werkzeuges durchgeführt. So erstreckt sich bei einer besonders bevorzugten Ausführungsform der Erfindung die Ausnehmung des Lagereinsatzes bis zu dessen Oberseite, um den Einsatz eines Werkzeuges von oben in die Ausnehmung des Lagereinsatzes und/oder die Ausnehmung der Tibiakomponente zu ermöglichen. Hierzu hat die Ausnehmung des Lagereinsatzes und ggf. der Tibiakomponente einen den Einsatz eines Werkzeuges zum Abwärts- oder Aufwärtsbewegen des Verbindungsteiles ermöglichenden Querschnitt. Ferner weist die Oberseite des Verbindungsteiles zweckmäßigerweise eine das Ansetzen eines Werkzeuges zum Abwärts- oder Aufwärtsbewegen des Verbindungsteiles ermöglichende Ausgestaltung auf. Beispielsweise kann die Oberseite des Verbindungsteiles einen Schlitz besitzen, in den ein von oben eingeführter Schraubendreher greifen kann, um eine Drehbewegung des Verbindungsteiles zu bewirken.

Um die Einführung des Werkzeuges von oben zu ermöglichen, weist zweckmäßigerweise auch die Femurkomponente eine entsprechende Ausnehmung auf.

Das Verbindungsteil selbst kann als Hohlteil ausgebildet sein oder einen Vollquerschnitt besitzen. Die letztgenannte Ausführungsform wird bevorzugt, um die Oberseite des Verbindungsteiles mit einer Ausgestaltung zu versehen, die das Ansetzen eines Werkzeuges ermöglicht.

Das Aufwärts- und Abwärtsbewegen des Verbindungsteiles wird vorzugsweise mit Hilfe einer Schraubbewegung realisiert. Hierzu sind Verbindungsteil und die Ausnehmung des Lagereinsatzes oder die Ausnehmung der Tibiakomponente mit entsprechenden Schraubgewinden versehen. Dabei weist das Verbindungsteil zweckmäßigerweise einen Drehlagerabschnitt und einen Befestigungsabschnitt zum Fixieren in der zugehörigen Ausnehmung auf. Dieser Befestigungsabschnitt ist dabei zweckmäßigerweise ein Gewindeabschnitt, wobei die zugehörige Ausnehmung einen passenden Gewindeabschnitt aufweist.

Bei der zuletzt beschriebenen Ausführungsform ergeben sich somit zwei bevorzugte Varianten. Bei der ersten Variante ist das Verbindungsteil von der Ausnehmung der Tibiakomponente aufgenommen und wird in seine Funktionsstellung nach oben aus der Tibiakomponente herausbewegt, so daß es von der Plattform vorsteht und das entsprechende Drehlager für den Lagereinsatz bildet. Bei dieser Ausführungsform hat das Verbindungsteil vorzugsweise einen oberen, glatt ausgebildeten Drehlagerabschnitt und einen unteren Gewindeabschnitt.

Bei der zweiten Variante ist das Verbindungsteil von der Ausnehmung im Lagereinsatz aufgenommen und wird in seine Funktionsstellung nach unten aus dem Lagereinsatz herausbewegt, so daß es in die Ausnehmung der Tibiakomponente eingreift und auf diese Weise das Drehlagerelement bildet.

Hierbei hat das Verbindungsteil vorzugsweise einen oberen Gewindeabschnitt und einen unteren Drehlagerabschnitt mit glatter Oberfläche.

In beiden Fällen wirken die Drehlagerabschnitte mit glatter Oberfläche mit entsprechenden glatt ausgebildeten Begrenzungswänden der Ausnehmungen zusammen.

Es versteht sich von selbst, daß das Verbindungsteil hierbei als zylindrischer Körper ausgebildet ist.

Die Ausbildung der Ausnehmung in der Tibiakomponente bereitet keine Probleme, da die Tibiakomponente normalerweise einen sich nach unten erstreckenden und sich verengenden Schaft zur Verankerung im Tibiaknochen (Unterschenkelknochen) aufweist. In diesen Schaft kann sich die Ausnehmung von der Tibiaplattform aus hinein erstrecken. In diese Ausnehmung kann das in Funktionsstellung sich von der Plattform nach oben und in die Ausnehmung des Lagereinsatzes hinein erstreckende Verbindungsteil ohne weiters versenkt werden.

Bei der anderen Ausführungsform der Erfindung wird das Verbindungsteil in seiner Stellung zum Austauschen des Lagereinsatzes von der Ausnehmung im Lagereinsatz aufgenommen, so daß es nicht von der Unterseite des Lagereinsatzes vorsteht. Normalerweise sollte das Verbindungsteil in dieser Stellung auch nicht über die Oberseite des Lagereinsatzes hinausragen. Dies kann jedoch der Fall sein, falls hierdurch keine Behinderung der Femurkomponente bewirkt wird.

Die Erfindung wird nachfolgend anhand von drei Ausführungsbeispielen in Verbindung mit der Zeichnung im einzelnen erläutert. Es zeigen:
- Figur 1: einen Vertikalschnitt durch eine Kniegelenkprothese einer ersten Ausführungsform der Erfindung im zusammengebauten Zustand in einer ersten Position des Verbindungsteiles;
- Figur 2: einen Vertikalschnitt durch eine Kniegelenkprothese einer ersten Ausführungsform der Erfindung im zusammengebauten Zustand in einer zweiten Position des Verbindungsteiles;
- Figur 3: einen Vertikalschnitt durch eine Kniegelenk-Prothese einer zweiten Ausführungsform der Erfindung im zusammengebauten Zustand in einer ersten Position des Verbindungsteiles;
- Figur 4: einen Vertikalschnitt durch eine Kniegelenk-Prothese einer zweiten Ausführungsform der Erfindung im zusammengebauten Zustand in einer zweiten Position des Verbindungsteiles; und
- Figur 5: einen Vertikalschnitt durch eine Kniegelenkprothese einer dritten Ausführungsform der Erfindung im zusammengebauten Zustand.

Die nachfolgenden Ausführungsbeispiele werden nur in Verbindung mit den für die Erfindung wichtigen Teilen erläutert. Weitere Teile, die für die Erfindung nur eine untergeordnete oder überhaupt keine Rolle spielen, werden nachfolgend nicht im einzelnen beschrieben. Diesbezüglich wird auf die bereits eingangs erwähnte EP 0 678 286 B1 verwiesen.

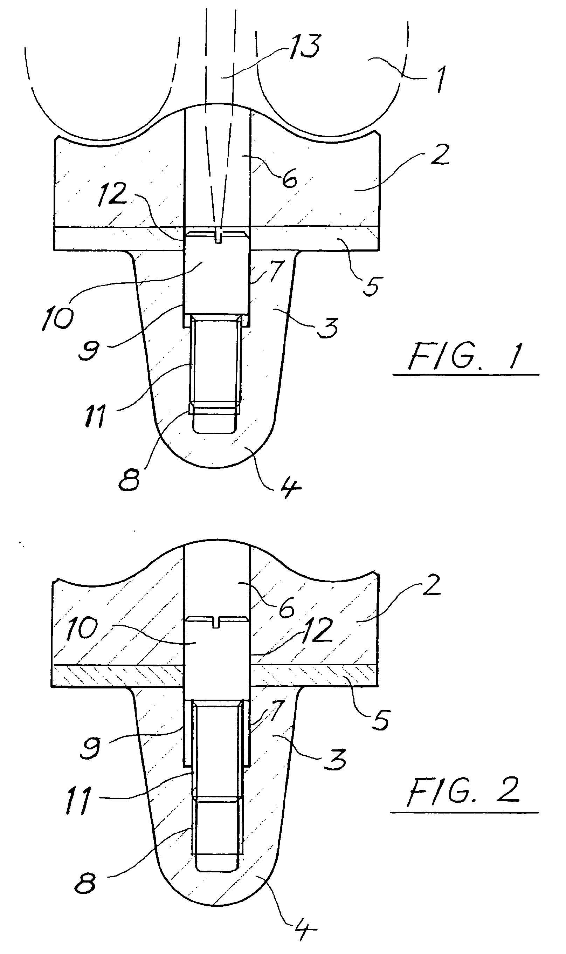

Figur 1 zeigt eine erste Ausführungsform einer implantierten Kniegelenkprothese. Die nur schematisch dargestellte Femurkomponente 1 ist auf der entsprechend gekrümmten Oberseite eines Lagereinsatzes 2 gelagert und kann auf dieser eine Rollbewegung durchführen. Der Lagereinsatz 2 ist drehbar auf der Plattform 5 einer Tibiakomponente 3 gelagert, die einen sich von der Plattform 5 in der Figur nach unten erstreckenden Schaft 4 aufweist. Im Schaft befindet sich eine Ausnehmung 7, die mittig angeordnet ist und sich durch die Plattform 5 bis zur Oberseite der Plattform erstreckt. In Verlängerung dieser Ausnehmung 7 ist eine Ausnehmung 6 im Lagereinsatz angeordnet, die sich von der Unterseite des Lagereinsatzes bis zu dessen Oberseite erstreckt.

Die Ausnehmung 7 in der Tibiakomponente weist einen in der Figur unteren Gewindeabschnitt 8 und einen darüber angeordneten glattwandigen Abschnitt 9 auf. In die Ausnehmung 7 mit zylindrischem Querschnitt ist ein zylindrisches Verbindungsteil 10 eingeschraubt, das einen in der Figur unteren Gewindeabschnitt 11 und oberen glattwandigen Abschnitt 12 besitzt. Der Gewindeabschnitt 11 kämmt mit dem entsprechenden Gewindeabschnitt 8 der Ausnehmung, während der glattwandige Abschnitt 12 mit dem glattwandigen Abschnitt 9 der Ausnehmung 7 in Kontakt steht. Das Verbindungsteil 10 ist so in der Ausnehmung 7 angeordnet, daß es mit seiner Oberseite etwa bündig mit der Oberseite der Tibiaplattform 5 abschließt. In dieser Stellung des Verbindungsteiles 10 kann somit der Lagereinsatz 2 in einer Richtung senkrecht zur Blattebene in Figur 1 aus der implantierten Prothese entfernt werden, ohne daß er dabei vom Verbindungsteil 10 behindert wird. In dieser Stellung übt das Verbindungsteil 10 keine Drehlagerfunktion für den Lagereinsatz 2 aus.

Figur 2 zeigt in einem entsprechenden Schnitt wie Figur 1 das Verbindungsteil 10 in einer Stellung, in der es eine Drehlagerfunktion für den Lagereinsatz 2 ausübt. Um das Verbindungsteil 10 aus der in Figur 1 gezeigten Stellung in die in Figur 2 gezeigte Stellung zu überführen, wird ein in Figur 1 schematisch dargestellter Schaubendreher 13 von oben durch die Ausnehmung 6 im Lagereinsatz 2 eingeführt und mit einem in der Oberseite des Verbindungsteiles 10 vorgesehenen Schlitz in Eingriff gebracht. Durch Drehen wird das Verbindungsteil 10 nunmehr aus der Ausnehmung 7 heraus nach oben bewegt, bis es die in Figur 2 dargestellte Endstellung erreicht hat. In dieser Stellung befindet sich der Gewindeabschnitt 11 des Verbindungsteiles noch teilweise mit dem Gewindeabschnitt 8 der Ausnehmung 7 in Eingriff, so daß das Verbindungsteil 10 noch sicher in der Tibiakomponente fixiert ist. Das Gewinde ist hierbei so ausgewählt, daß es einen hohen Selbsthemmungseffekt besitzt, so daß das Verbindungsteil 10 nicht ohne weiteres in der Ausnehmung 7 verdreht werden kann. In der in Figur 2 gezeigten Endstellung ragt der glattwandige Abschnitt 12 des Verbindungsteiles 10 in die glattwandig ausgebildete Ausnehmung 6 des Lagereinsatzes hinein und bildet auf diese Weise ein Drehlager für den Lagereinsatz 2. Dieser kann sich somit funktionsgerecht relativ zur Plattform 5 drehen. Soll der Lagereinsatz 2 ausgetauscht werden, wird das Verbindungsteil 10 wiederum mit einem geeigneten Schraubendreher 13 nach unten in die Ausnehmung 7 versenkt.

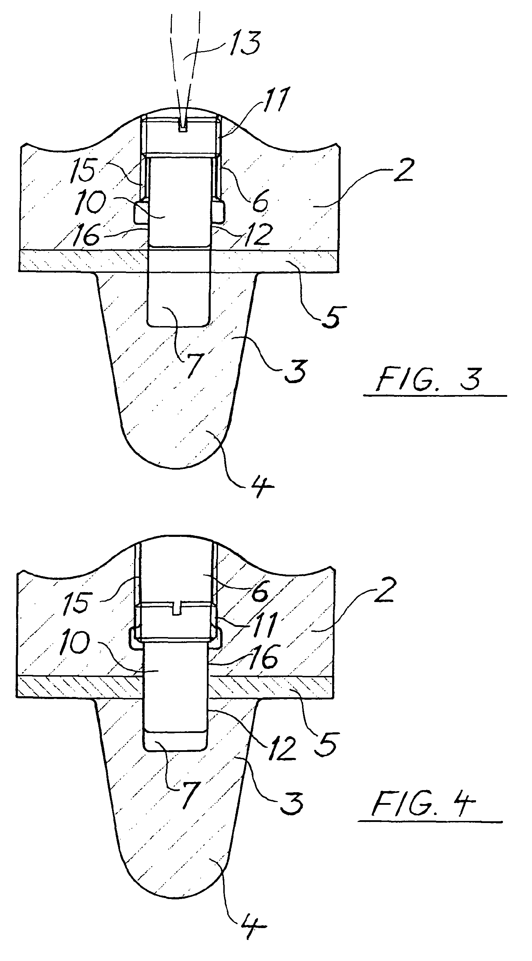

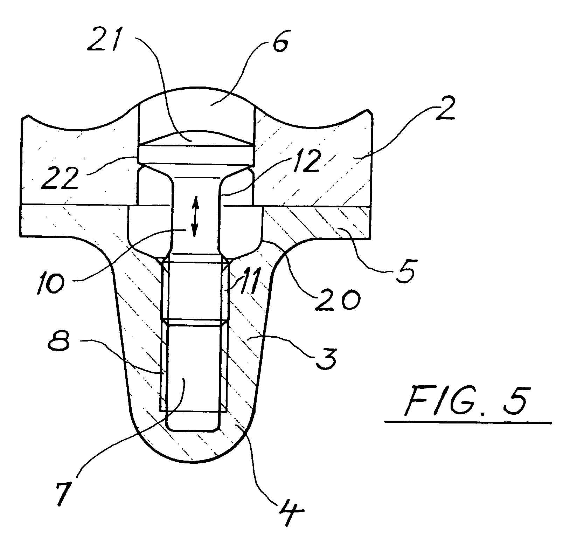

Die Figuren 3 und 4 zeigen eine zweite Ausführungsform der Erfindung, die sich von der Ausführungsform der Figuren 1 und 2 im wesentlichen nur dadurch unterscheidet, daß hierbei das Verbindungsteil 10, wenn es nicht in Funktion ist, im Lagereinsatz 2 und nicht wie bei der Ausführungsform der Figuren 1 und 2 in der Tibiakomponente aufgenommen ist. Figur 3 zeigt, daß die zentrale Ausnehmung 6 im Lagereinsatz 2 einen unteren glattwandigen Abschnitt 16 und einen oberen Gewindeabschnitt 15 aufweist. In Verlängerung dieser Ausnehmung 6 erstreckt sich eine im Vergleich zur Ausführungsform der Figuren 1 und 2 kürzere Ausnehmung 7 von der Oberseite der Plattform 5 in den Schaft 4 der Tibiakomponente 3 hinein. Diese Ausnehmung 7 ist glattwandig ausgebildet.

Figur 3 zeigt, daß der Gewindeabschnitt 11 des Verbindunsgteiles 10 mit dem Gewindeabschnitt 15 der Ausnehmung 6 verschraubt ist und daß der glattwandige Abschnitt 12 des Verbindungsteiles 10 mit dem glattwandigen Abschnitt 16 der Ausnehmung 6 in Kontakt steht. Die Unterseite des Verbindungsteiles 10 schließt etwa bündig mit der Unterseite des Lagereinsatzes 2 ab, so daß in der in Figur 3 gezeigten Stellung des Verbindungsteiles 10 der Lagereinsatz 2 in einer Richtung senkrecht zur Blattebene der Figur 3 aus der implantierten Prothese entfernt werden kann, wobei der Lagereinsatz das Verbindungsteil 10 in der Ausnehmung 6 enthält.

Um das Verbindungsteil 10 in seine Funktionstellung zu bewegen, wird es mit einem schematisch bei 13 gezeigten Schraubendreher in der Figur nach unten geschraubt, so daß der glattwandige Abschnitt 16 mit der glattwandigen Ausnehmung 7 in der Tibiakomponente in Eingriff tritt. Diese Stellung ist in Figur 4 gezeigt. In dieser Stellung kämmt der Gewindeabschnitt 11 des Verbindungsteiles 10 noch etwa mit der Hälfte des Gewindeabschnittes 15 der Ausnehmung 6. Der glattwandige Abschnitt 12 steht soweit in die Ausnehmung 7 vor, daß er einen nach unten vorstehenden Drehzapfen für den Lagereinsatz 2 bildet, so daß der Lagereinsatz 2 zusammen mit dem Verbindungsteil 10 relativ zur Tibiakomponente gedreht werden kann. Soll der Lagereinsatz 2 ausgetauscht werden, wird das Verbindungsteil 10 mit Hilfe des Schraubendrehers nach oben in die in Figur 3 gezeigte Stellung bewegt, wonach der Lagereinsatz 2 entfernt werden kann.

Figur 5 zeigt eine dritte Ausführungsform einer Kniegelenkprothese im zusammengebauten Zustand. Wie bei den bisher beschriebenen Ausführungsformen besitzt die Kniegelenkprothese eine Femurkomponente (nicht gezeigt), die auf der entsprechend gekrümmten Oberseite eines Lagereinsatzes 2 gelagert ist und auf dieser eine Rollbewegung durchführen kann. Der Lagereinsatz 2 ist drehbar auf der Plattform 5 einer Tibiakomponente 3 gelagert, die einen sich von der Plattform 5 in der Figur nach unten erstreckenden Schaft 4 aufweist. Im Schaft befindet sich eine Ausnehmung 7, die mittig angeordnet ist und am oberen Ende in eine erweiterte Ausnehmung 20 übergeht, die sich bis zur Oberseite der Plattform erstreckt. Etwa in Verlängerung dieser erweiterten Ausnehmung 20 ist eine Ausnehmung 6 im Lagereinsatz angeordnet, die sich von der Unterseite des Lagereinsatzes bis zu dessen Oberseite erstreckt.

Die Ausnehmung 7 in der Tibiakomponente weist einen sich bis zum oberen Ende derselben erstreckenden Gewindeabschnitt 8 auf. Die Wandung der sich daran anschließenden erweiterten Ausnehmung 20 ist glatt ausgebildet.

In die Ausnehmung 7 ist ein zylindrisches Verbindungsteil 10 mit erweitertem Kopf 21 eingeschraubt. Das Verbindungsteil 10 kann so weit in die Ausnehmung 7 eingeschraubt werden, daß dessen erweiterter Kopf 21 von der Ausnehmung 20 aufgenommen wird, so daß der Kopf 21 des Verbindungsteiles in dieser Position etwa bündig mit der Oberseite der Plattform 5 abschließt. Auf diese Weise kann der Lagereinsatz 2 ohne weiteres entfernt werden. Wird das Verbindungsteil 10 aus der Ausnehmung 7 herausgeschraubt, etwa in die in Figur 5 gezeigte Stellung, bildet der Kopf 21 ein Drehlager für den Lagereinsatz 2 in entsprechender Weise wie bei den vorstehend beschriebenen Ausführungsformen. Hierbei steht die seitliche Fläche 22 des Kopfes 21 mit der Innenwandung der Ausnehmung 6 des Lagereinsatzes 2 in Gleiteingriff. Das Aus- und Einschrauben des Verbindungsteiles 10 kann wie bei den vorstehend beschriebenen Ausführungsformen mit Hilfe eines geeigneten Werkzeuges erfolgen, das an der Oberseite des Kopfes 21 des Verbindungsteiles 10 ansetzt. Auf dieser Oberseite kann sich eine entsprechende Vertiefung zum Angriff des Werkzeuges befinden, die in Figur 5 nicht gesondert dargestellt ist.

Damit ein Schraubeingriff des Verbindungsteiles 10 mit der Ausnehmung 7 möglich ist, weist dessen zylindrischer Schaft einen entsprechenden Gewindeabschnitt auf, der bei 11 gezeigt ist.

## Patentansprüche

1. Kniegelenkprothese mit einer Femurkomponente (1) einer Tibiakomponente (3) mit einer Plattform (5) und einem zwischen der Femur- und Tibiakomponente (1,3) angeordneten Lagereinsatz (2),
wobei zwischen der Plattform (5) der Tibiakomponente (3) und dem Lagereinsatz (2) ein Verbindungsteil (10) zur drehbaren Lagerung des Lagereinsatzes (2) relativ zur Plattform (5) vorgesehen ist,
Tibiakomponente (3) und Lagereinsatz (2) je eine Ausnehmung (7,6) zur Aufnahme des Verbindungsteiles (10) besitzen und
das Verbindungsteil (10) aus einer von der Ausnehmung (7,6) der Tibiakomponente (3) oder des Lagereinsatzes (2) aufgenommenen Stellung in eine in die andere Ausnehmung (6,7) hineinragende und ein Drehlagerelement für den Lagereinsatz (2) bildende Stellung und wieder zurück bewegbar ist.

2. Kniegelenkprothese nach Anspruch 1, **dadurch gekennzeichnet, daß** sich die Ausnehmung (6) des Lagereinsatzes bis zu dessen Oberseite erstreckt.

3. Kniegelenkprothese nach Anspruch 2, **dadurch gekennzeichnet, daß** die Ausnehmung (6) des Lagereinsatzes (2) und ggf. der Tibiakomponente (3) einen den Einsatz eines Werkzeuges zum Abwärts- oder Aufwärtsbewegen des Verbindungsteiles (10) ermöglichenden Querschnitt besitzt.

4. Kniegelenkprothese nach Anspruch 2 oder 3, **dadurch gekennzeichnet, daß** die Oberseite des Verbindungsteiles (10) eine das Ansetzen eines Werkzeuges zum Abwärtsoder Aufwärtsbewegen des Verbindungsteiles (10) ermöglichende Ausgestaltung aufweist.

5. Kniegelenkprothese nach einem der vorangehenden Ansprüche, **dadurch gekennzeichnet, daß** das Verbindungsteil (10) einen Vollquerschnitt aufweist.

6. Kniegelenkprothese nach einem der vorangehenden Ansprüche, **dadurch gekennzeichnet, daß** das Verbindungsteil (10) einen Drehlagerabschnitt (12) und einen Befestigungsabschnitt (11) zum Fixieren in der zugehörigen Ausnehmung (6,7) aufweist.

7. Kniegelenkprothese nach Anspruch 6, **dadurch gekennzeichnet, daß** der Befestigungsabschnitt (11) ein Gewindeabschnitt ist und daß die zugehörige Ausnehmung (6,7) einen passenden Gewindeabschnitt (15,8) aufweist.

8. Kniegelenkprothese nach einem der vorangehenden Ansprüche, **dadurch gekennzeichnet, daß** das Verbindungsteil (10) einen erweiterten Kopf (21) und die Plattform (5) eine Ausnehmung (20) zur Aufnahme des Kopfes (21) aufweist.
